Europäisches Patentamt

European Patent Office

Office européen des brevets

(19)

(11) Veröffentlichungsnummer : **0 441 799 B1**

(12) **EUROPÄISCHE PATENTSCHRIFT**

(45) Veröffentlichungstag der Patentschrift :
19.08.92 Patentblatt 92/34

(51) Int. Cl.⁵ : **A61M 5/158**

(21) Anmeldenummer : 89910847.6

(22) Anmeldetag : 22.09.89

(86) Internationale Anmeldenummer :
PCT/EP89/01102

(87) Internationale Veröffentlichungsnummer :
WO 90/03195 05.04.90 Gazette 90/08

(54) **KANÜLE INSBESONDERE FÜR DIE GEFÄSSPUNKTION.**

(30) Priorität : 24.09.88 DE 8812099 U

(43) Veröffentlichungstag der Anmeldung :
21.08.91 Patentblatt 91/34

(45) Bekanntmachung des Hinweises auf die
Patenterteilung :
19.08.92 Patentblatt 92/34

(84) Benannte Vertragsstaaten :
BE DE FR GB IT NL

(56) Entgegenhaltungen :
EP-A- 0 164 600
EP-A- 0 253 990
GB-A- 2 088 721
US-A- 4 627 842
US-A- 4 676 783

(73) Patentinhaber : **SÜDDEUTSCHE FEINMECHANIK GMBH
Brückenstrasse
W-6480 Wächtersbach 1 (DE)**

(72) Erfinder : **KÄTHNER, Volkard
Haitzerstrasse 4
W-6480 Wächtersbach (DE)**
Erfinder : **VOGEL, Dieter
Bahnhofstrasse 36
W-6497 Steinau (DE)**

(74) Vertreter : **Stoffregen, Hans-Herbert, Dr.
Dipl.-Phys.
Patentanwälte Strasse & Stoffregen
Salzstrasse 11a Postfach 2144
W-6450 Hanau/Main 1 (DE)**

EP 0 441 799 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung bezieht sich auf eine Kanüle nach dem Oberbegriff des Anspruchs 1.

Entsprechende Kanülen werden z.B. bei der Punktion von Körperhöhlen und Blutgefäßen benutzt, um z.B. Blut zu entnehmen oder Blut oder andere Flüssigkeiten einzubringen. Hierbei ist es häufig erforderlich, daß die Kanüle z.B. bei einer Dialysebehandlung längere Zeit in dem Blutgefäß verbleibt. Dabei muß sichergestellt sein, daß die Kanülenöffnung nicht von der Wandung des Blutgefäßes verschlossen wird. Um dies zu verhindern bzw. optimale Durchflußbedingungen zu ermöglichen, ist die Kanüle gegenüber der Handhabe, die z.B. als Flügel ausgebildet und auf der Hautoberfläche mittels eines Pflasters befestigt ist, drehbar. Da der auf die Kanülenhalterung aufgeschobene Schlauch normalerweise die Handhabe mehr oder weniger berührt, liegen beim Verdrehen der Kanüle grundsätzliche unterschiedliche Drehmomente vor, die häufig ein genaues Einstellen erschweren bzw. die Gefahr von Verletzungen der Blutgefäße in sich bergen. Aus der EP-A-0 253 990 ist eine Venenkanüle bekannt, die neben einer Handhabe ein Befestigungsstück aufweist, mittels dessen ein Fixieren auf einer Hautpartie möglich ist. Da Handhabe und Befestigungsstück unabhängig zueinander drehbar sind, liegen grundsätzlich stets unterschiedliche Bedingungen beim Hantieren der Venenkanüle vor.

Aufgabe der vorliegenden Erfindung ist es unter anderem, eine Kanüle der eingangs genannten Art so auszubilden, daß stets gleiche Bedingungen beim Verdrehen der Kanüle vorliegen, so daß gewährleistet ist, daß die Kanülenöffzung problem- und gefahrlos in einem Blutgefäß gedreht werden kann. Auch ist es Aufgabe, daß die Handhabe in einer eindeutig definierten Position auf dem Kanülenhalter angeordnet ist. Ferner soll sichergestellt sein, daß die Handhabe zu der Kanülenhalterung in radialer Hinsicht eine gewünschte Orientierung einnimmt. Schließlich sollen Möglichkeiten gegeben sein, durch die ein sicheres Aufnehmen einer gebrauchten Nadel von der Schutzkappe möglich ist.

Die Aufgabe wird erfindungsgemäß im wesentlichen dadurch gelöst, daß die Schutzkappe und die Handhabe als Einheit von der Flügelspitze her auf den Kanülenhalter schiebbar sind, daß der Kanülenhalter einen Rastbund und die Handhabe eine diesem zugeordnete Rastnut aufweisen und daß bei auf dem Kanülenhalter angeordneter Einheit der Rastbund in der Rastnut verrastet ist, und daß der Kanülenhalter schlauchseitig einen Absatz aufweist.

Weitere Lösungsvorschläge ergeben sich aus den Ansprüchen 2 bis 9.

Weitere Einzelheiten, Vorteile und Merkmale der Erfindung ergeben sich nicht nur aus den Ansprüchen, den diesen zu entnehmenden Merkmalen -für sich und/oder in Kombination-, sondern auch aus den der Zeichnung zu entnehmenden bevorzugten Ausführungsbeispielen, die wesentliche Merkmale der Erfindung erkennen lassen.

Es zeigen:

Fig. 1 eine perspektivische Darstellung einer Kanüle,

Fig. 2 eine Schnittdarstellung durch eine Kanüle,

Fig. 3 eine Schnittdarstellung durch die Kanüle gemäß Fig. 2, jedoch um 90° gedreht,

Fig. 4 eine Detaildarstellung der Kanülen gemäß Fig. 2 und 3,

Fig. 5 einen Ausschnitt aus einem weiteren Ausführungsbeispiel einer Kanüle,

Fig. 6 einen im Vergleich zur Darstellung der Fig. 5 um 90° gedrehten Ausschnitt der Kanüle,

Fig. 7 ein Ausführungsbeispiel einer Kanüle mit Schutzkappe in Schnittdarstellung,

Fig. 8 eine hervorzuhebende Ausführungsform einer Kanüle mit Schutzkappe,

Fig. 9 und 10 weitere Ausschnitte von Ausführungsbeispielen einer Kanüle und

Fig. 11 eine weitere Ausführungsform einer erfindungsgemäßen Kanüle.

In den Figuren, in denen gleiche Elemente mit gleichen Bezugszeichen versehen sind, sind verschiedene Ausführungsformen von Kanülen insbesondere für die Gefäßpunktion für z.B. die extrakorporale Blutbehandlung dargestellt, die als wesentliche Elemente eine Kanüle oder Nadel (12) mit schräg angeschliffener Spitze (14), einen die Kanüle in ihrem hinteren Bereich umgebende Kanülenhalterung (16), eine vorzugsweise flügelartig ausgebildete Handhabe (18) sowie eine Schutzkappe (20) umfaßt. Die Kanülenhalterung (16) kann auf die Kanüle (12) gespritzt, geklebt, geschrumpft oder ähnlich angeordnet sein, wodurch eine Unverrutschbarkeit gewährleistet sein soll. Die Handhabe (18) weist vorzugsweise zwei von einen zylindrischen die Kanülenhalterung (16) umgebenden Abschnitt (22) ausgehende Flügel (24) und (26) auf, die nach Punktieren eines Gefäßes auf der Haut mittels z.B. eines Klebstreifens befestigt werden, um so eine Unverrutschbarkeit zu gewährleisten.

Die Schutzkappe (20) ist vorzugsweise über eine Sollbruchstelle (28) mit der Handhabe (18) verbunden. Die Schutzkappe (28) weist in Bereich der Sollbruchstelle einen senkrecht zur Längsachse der Schutzkappe (20) verlaufenden Prallschutz in Form von gegebenenfalls einer flanschartigen Erweiterung (30) auf.

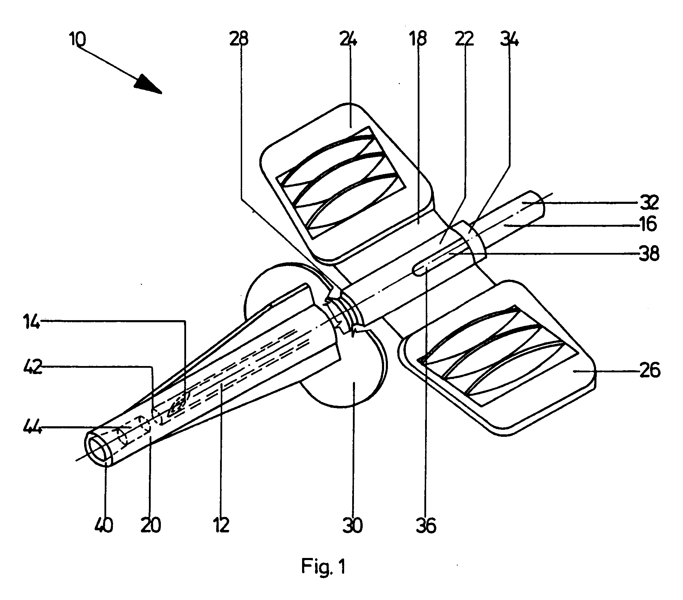

Auf den rückwärtigen Abschnitt (32) der Kanülenhalterung (16) wird ein nicht dargestellter Schlauch geschoben. Damit dieser eine Drehung der Handhabe (18) nicht unkontrolliert beeinflussen kann, wodurch sich unterschiedliche, die Handhabbarkeit beeinträchtigende Drehmomente ergeben könnten, ist erfindungsgemäß vorgesehen, daß der Schlauch in definierten Abstand zu der Handhabe (18) den Abschnitt (32) umgibt. Hierzu ist ein Absatz (34) vorgesehen, der für die Stirnfläche des Schlauches als Anschlag dient. Der Absatz (34) kann als Flansch ausgebildet sein, so daß auch die Handhabe (18) an der den Schlauch gegenüberliegenden Fläche des Absatzes (34) anlegbar ist. Um eine Unverdrehbarkeit der Handhabe (18) in bezug auf die Kanülenhalterung (16) und damit auf die Kanüle (12) zu erzielen, ist eine Verrastung in z.B. einer Nut-Feder-Verbindung vorgesehen, die im Ausführungsbeispiel der Kanüle (10) nach Fig. 1 dadurch erzielt wird, das in Längsrichtung der Kanülenhalterung (16) ein linienförmiger Vorsprung (38) in Richtung des zylinerischen Abschnitts (22) der Handhabe (18) ausgeht, der in eine entsprechende Aussparung (36) in der Handhabe (18) eingreift. Hierdurch ist eine radiale Verdrehsicherung gewährleistet.

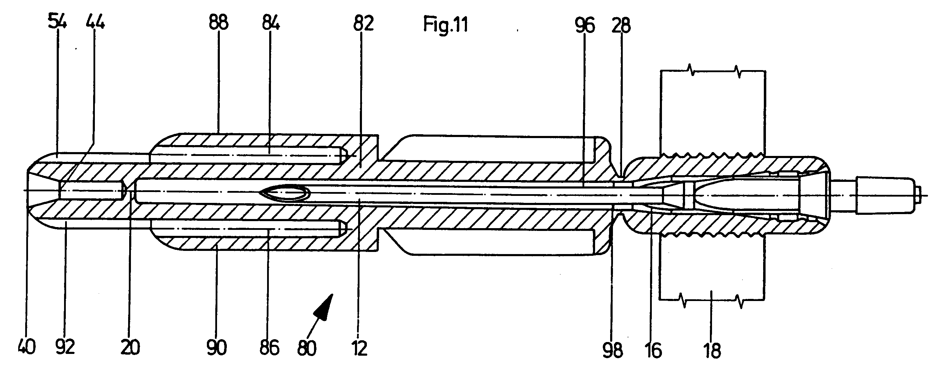

Die Schutzkappe (20) weist im Abstand zu ihrem Stirnbereich (40) eine Membran (42) auf, die bei unbenutzter Kanüle sicherstellt, daß diese hinreichend steril bleibt, also im erforderlichen Umfang geschützt ist. Zwischen der Mebran (42) und dem Stirnbereich (40) ergibt sich ein trichterförmiges Einführungsteil (44), in das eine benutzte Kanüle einbringbar ist, um sodann die Membran (42) zu durchstechen. Durch eine entsprechende Konstruktion ist gewährleistet, daß eine benutzte Kanüle erneut sicher von der Schutzkappe (20) aufgenommen wird. Andere Möglichkeiten der Aufnahme der Nadel werden an Hand der Fig. 11 erläutert.

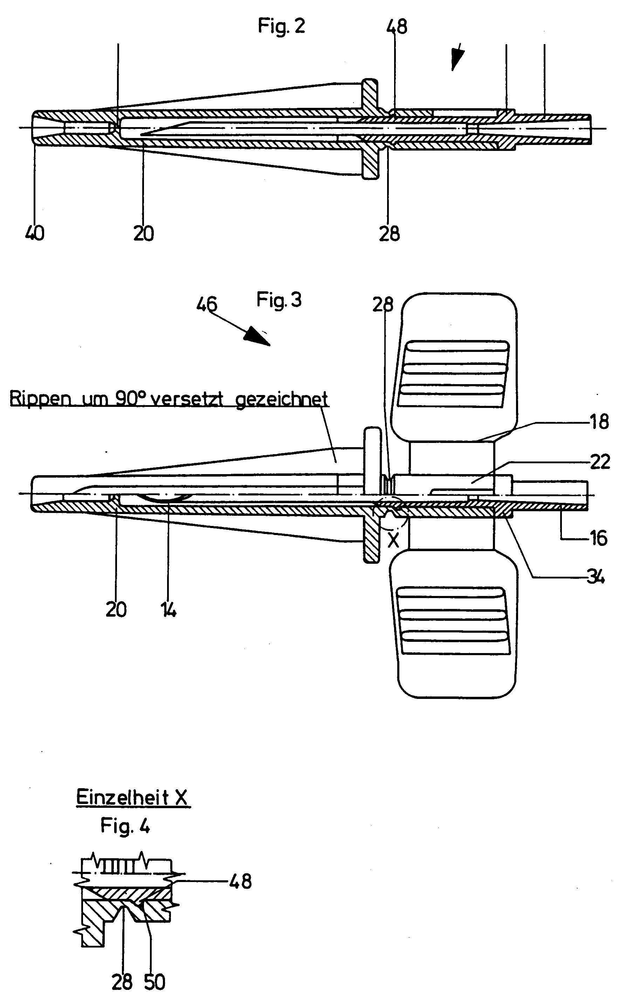

In den Fig. 2 bis 4 ist eine dem Prinzip her der Fig. 1 entsprechende Kanüle (46) dargestellt, durch die eine axiale Unverrückbarkeit von Handhabe (18) zu Kanülenhalterung (16) ergibt. So weist die Kanülenhalterung (16) einen umlaufenden Rastbund (48) auf, dem eine Rastnut (50) in der Innenwandung des zylinerischen Abschnitts (22) der Handhabe (18) zugeordnet ist. Beim Zusammensetzen der Kanüle muß demzufolge die Handhabe mit vorzugsweise angespritzter Schutzkappe (20) nur von der Kanülenspitzen (14) her auf die Kanülenhalterung (16) in einem Umfang geschoben werden, bis eine Verrastung zwischen dem Bund (48) und der Nut (50) erfolgt. Eine Verdrehsicherung kann zusätzlich durch die Nut (36) und Vorsprung (38) von der Handhabe (18) und Kanülenhalterung (16) gemäß Fig. 1 vorgenommen werden.

Den Fig. 2 bis 4 ist des weiteren die Sollbruchstelle (28) klar zu entnehmen, um bei Benutzung der Kanüle die Schutzkappe (20) schnell und sicher entfernen zu können. Auch erkennt man die im Abstand zum Stirnbereich (40) verlaufende Membran (42), die eine Stärke von vorzugsweise weniger als 1 mm aufweist.

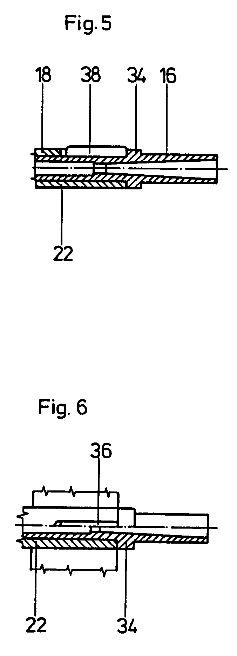

Die Fig. 5 und 6 zeigen Ausschnitte der Kanüle im Bereich der Verbindung zwischen Kanülenhalterung (16) und Handhabe (18). Man erkennt den umlaufenden Absatz (34), dessen Außenfläche, also in der Zeichnung dessen rechte Seite als beschlag für einen nicht dargestellten Schlauch und innenseitig, also links als Anschlag für die Stirnfläche des zylinderischen Abschnitts (22) dient. Ferner weist der Abschnitt (22) die Längsnut (36) auf, in die der Vorsprung (38) zur Verdrehsicherung der Handhabe (18) eingreift.

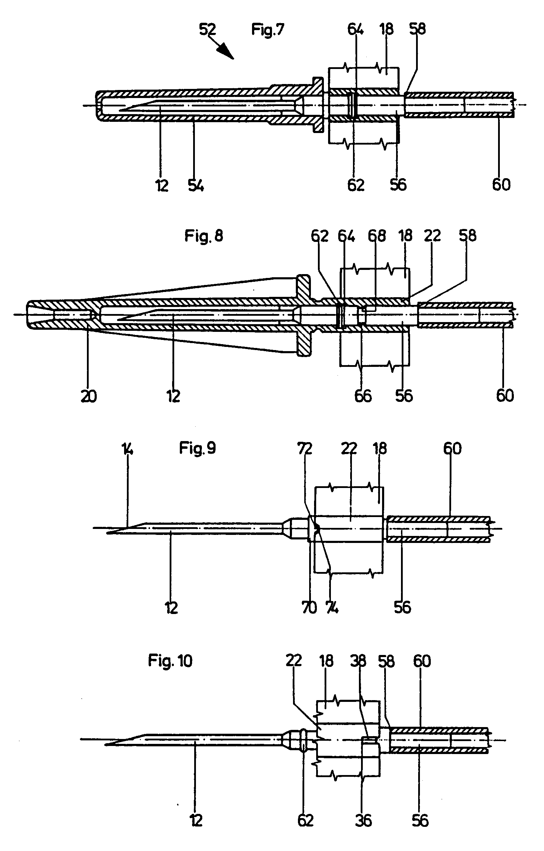

In Fig. 7 ist ein Ausschnitt einer Kanüle (52) gezeichnet, bei der eine Handhabe (54) als getrenntes Teil zu der Handhabe (18) ausgebildet ist bestelle des umlaufenden scheibenförmigen Vorsprungs (34) als doppelseitiger Anschlag für Schlauch und Handhabe gemäß der Fig. 1 bis 6 ist die Kanülenhalterung (56) mit einer Stufe (58) versehen, die einen Anschlag für einen Schlauch (60) bildet. Die Handhabe (18) wird mit der Kanülenhalterung (56) mittels eines von diesem ausgehenden Rastbundes (62) verbunden, der in eine entsprechend zugeordnete umlaufende Nut (64) bei aufgedrückter Handhabe (18) einrastet.

Das Ausführungsbeispiel der Fig. 8 weist neben dem Rastbund (62) und der Rastnut (60) eine an der Innenfläche des zylinderischen Abschnitts (22) der Handhabe (18) umlaufenden Vorsprung (66) auf, der in eine in der Kanülenhalterung (56) eingelassene Nut (68) eingreift. Es folgt quasi eine Doppelrastung zwischen der Kanülenhalterung (56) und der Handhabe (18). Die Verbindung zwischen der Handhabe (18) und der Schutzkappe (20) und dessen Ausbildung entspricht der der Fig. 1.

In Fig. 9 ist eine Möglichkeit einer rastbaren Verdrehbarkeit zwischen der Handhabe (18) und der Kanülenhalterung (56) dargestellt, um die Handhabe (18) in gewünschten Positionen zu der Kanülenhalterung festzulegen. So weist die Kanülenhalterung (56) einen umlaufenden bundartigen Vorsprung (70) auf, von dem in axialer Richtung und in Richtung der Handhabe sich erstrekkende Rastnocken (72) ausgehen, die ihrerseits in entsprechende Rastnuten (60) in dem zylinderischen Abschnitt (22) der Handhabe (18) eingreifen.

Nach der Fig. 10 ist der zylinderische Abschnitt (22) und damit die Handhabe (18) zum einen über den Rastbund (62) und zum anderen über den Vorsprung (38) und der Nut (36) verbunden. Durch den Rastbund ergibt sich folglich eine Unverschiebbarkeit in axialer Richtung. Durch das Wechselwirken von der in dem zylinderischen Abschnitt (22) vorhandenen Nut (36) und dem von der Kanülenhalterung (56) in axialer Richtung sich erstreckenden Vorsprung (28) ist eine Verdrehsicherung gegeben.

In Fig. 11 ist eine weitere Ausführungsform einer erfindungsgemäßen Kanüle (80) mit einer Schutzkappe (82) dargestellt, um weitere Möglichkeiten des Zurücksteckens der Nadel (12) in die Schutzkappe zu bieten. Eine Möglichkelt ist die bereits im Zusammenhang mit den Fig. 1, 2 und 3 beschriebene Membran (20), die im Abstand zu der Stirnfläche (40) der Schutzkappe (82) verläuft. Zwischen der Membran (20) und der Stirnfläche (40) erstreckt sich eine trichterförmige Öffnung (44), um das Einführen der Nadel (12) zu erleichtern.

Eine weitere Sicherungsmöglichkeit zum Befestigen einer gebrauchten Nadel an der Kappe (82) ist durch in Längsrichtung der Kappe (82) verlaufende zylinderische Aussparungen (84) und (86) gegeben, die im Durchmesser der Nadel (12) angepaßt sind und die in von der Außenseite der Schutzkappe (82) ausgehende Ausbuchtungen (88) und (90) verlaufen. Diese bilden mit den Aufnahmen (84) und (86) quasi Taschen für aufzunehmende gebrauchte Nadeln (12). Um das Einbringen in die Aussparungen (84) und (86) zu erleichtern, verlaufen vorzugsweise vom Stirnbereich (40) ausgehend in der Außenwandung der Schutzkappe (82) rinnenförmige Vertiefungen (92) und (94), die in den Aussparungen (84) und (86) münden und in diese bündig übergehen. Hierdurch wird erkennbar das Einbringen von gebrauchten Nadeln (12) in die Taschen (84) und (86) erleichtert.

Eine weitere Möglichkeit, um eine gebrauchte Nadel (12) wieder in die Schutzkappe einzubringen, ist dadurch gegeben, daß sich die Kanülenhalterung (16) mit ihrer Stirnfläche (96) vor der Sollbruchstelle (28) zwischen Schutzkappe (82) und Handhabe (18) erstreckt, so daß die Schutzkappe erneut auf die Nadel (12) gesteckt und auf den Absatz (98) der Kanülenhalterung (16) geschoben werden kann, der außerhalb der Handhabe (18) die Nadel (12) umgibt.

Vorzugsweise sind die Membran (20), die Aufnahmetaschen (84) und (86) sowie der Aufnahmeabschnitt (98) bei einer erfindungsgemäßen Kanüle realisiert, um so verschiedene Möglichkeiten des sicheren Aufbewahrens einer gebrauchten Nadel (12) in der Schutzkappe zu gewährleisten.

**Patentansprüche**

1. Kanüle (10, 46, 52, 80) insbesondere für die Gefäßpunktion wie für extrakorporale Blutbehandlung wie Dialyse oder Plasmaphorese mit schräg angeschliffener Kanülenspitze (14) und einem die Kanüle (12) aufnehmenden Kanülenhalter (16, 56), auf den rückwärtig ein Schlauch (60) aufschiebbar ist und der zumindest bereichsweise von einer vorzugsweise flügelartig ausgebildeten Handhabe (18) umgeben ist, und mit einer die Kanülenspitze umgebenden Schutzkappe (20),
**dadurch gekennzeichnet,**
daß die Schutzkappe (20) und die Handhabe (18) als Einheit von der Flügelspitze (14) her auf den Kanülenhalter (16, 56) schiebbar sind, daß der Kanülenhalter einen Rastbund (48, 62) und die Handhabe eine diesem zugeordnete Rastnut (50, 64) aufweisen und daß bei auf dem Kanülenhalter angeordneter Einheit der Rastbund in der Rastnut verrastet ist, und daß der Kanülenhalter (16, 56) schlauchseitig einen Absatz (34, 58) aufweist.

2. Kanüle nach Anspruch 1,
**dadurch gekennzeichnet,**
daß der Kanülenhalter (16) im Abstand zu seinem rückwärtigen Ende einen Flansch (34) aufweist, gegen den der Schlauch anlegbar ist.

3. Kanüle nach Anspruch 2,
**dadurch gekennzeichnet,**
daß der den Kanülenhalter (16) umgebende Bereich (22) der Handhabe (18) an der dem Schlauch abgewandten Fläche des Flansches (34) anliegt.

4. Kanüle nach zumindest einem der vorhergehenden Ansprüche,
**dadurch gekennzeichnet,**
daß der Kanülenhalter (16, 56) und/oder die Handhabe (18) bzw. deren den Kanülenhalter umgebender Abschnitt (22) Rastvorsprünge bzw. Rastausnehmungen (36, 38, 62, 64, 66, 68, 48, 50) aufweisen.

5. Kanüle nach zumindest einem der vorhergehenden Ansprüche,
**dadurch gekennzeichnet,**
daß die Handhabe (18) an einem von dem Kanülenhalter (56) vorzugsweise in dem der Kanülenspitze (40) zugewandten Bereich ausgehenden Anschlag (70) anliegt und daß in axialer Richtung des Kanülenhalters (56) von dem Anschlag Rastnocken (72) und/oder Rastaussparungen ausgehen, die mit von dem den Kanülenhalter (56) umgebenden stirnseitigen Bereich (22) der Handhabe (18) ausgehenden Rastaussparungen (74) und/oder Rastnasen wechselwirken.

6. Kanüle nach zumindest einem der vorhergehenden Ansprüche,
**dadurch gekennzeichnet,**
daß die Handhabe (18) zur Verdrehsicherung gegenüber der Kanülenhalterung (16) eine axial verlaufende linienförmige Aussparung (36) aufweist, in die zumindest bereichsweise ein von dem Kanülenhalter (16) ausgehender angepaßter Vorsprung (38) eingreift.

7. Kanüle nach vorzugsweise einem der vorhergehenden Ansprüche,
**dadurch gekennzeichnet,**

daß die Schutzkappe (82) außenwandseitig zumindest eine axial verlaufende hohlzylinderförmige Aüfnahme (84, 86) zur Wiederaufnahme der Kanüle (12) aufweist.

8. Kanüle nach Anspruch 7,
**dadurch gekennzeichnet,**
daß vom Stirnbereich (40) der Schutzkappe (82) ausgehend sich zumindest eine rinnenförmige Vertiefung (92, 94) erstreckt, die in der Aufnahme (84, 86) mündet.

9. Kanüle nach zumindest einem der vorhergehenden Ansprüche,
**dadurch gekennzeichnet,**
daß der Kanülenhalter (16) mit einem sich in Richtung der Kanülenspitze sich erstreckenden beschnitt (98) versehen ist, der von der Kanülenspitze aus betrachtet vor der Sollbruchstelle (28) und der Handhabe (18) verläuft.

**Claims**

1. Cannula (10, 46, 52, 80), in particular for puncturing blood vessels, for example for extacorporeal blood therapy such as dialysis or plasmaphoresis, comprising a beveled cannula tip (14) and a cannula holder (16, 56), wherein the cannula (12) is mounted, the cannula holder having at its ear end a flexible tube (60) and beeing at least partially surrounded by a wing-shaped handle (18) and a protector cap (20) covering the cannula tip,
**wherein**
the said protector cap (20) and the said handle (18) are formed as a unit, which is slipped onto the said cannula holder (16, 56) from the cannula tip (14) , the said cannula holder comprises a locking collar (48, 62) and the said handle comprises a snap-in groove (50, 64) whereby said locking collar is locked in said snap-in groove when the unit is mounted on the cannula holder and the said cannula holder (16, 56) is provided with a shoulder (34, 58) facing said tube.

2. Cannula according to claim 1
**wherein**
the said cannula holder (16) is equipped with a flange (34) which is provided at a certain distance from the rear end of the cannula holder and which is contacted by the flexible tube.

3. Cannula according to claim 2,
**wherein**
the said portion (22) of the said handle ( 18) surrounding the said cannula holder (16) rests against the said flange (34) on its face opposite the flexible tube.

4. Cannula according to at least any of the preceding claims,
**wherein**
the said cannula holder (16, 56) and/or the said handle (18) and/or its said portion (22) surrounding the cannula holder are provided with detent projections and detent recesses (36, 38, 62, 64, 66, 68, 48, 50) respectively.

5. Cannula according to at least any of the preceding claims,
**wherein**
the said handle (18) is in contact with a stop (70) extending from the cannula holder (56), preferably in the area facing the cannula tip (40), and that detent pins (72) and/or snap-in recesses extending from the said stop, in axial direction of the said cannula holder (56), are provided to interact with the detent recesses (74) and/or detent lugs extending from the said end portion (22) of the said handle (18) which surrounds the said cannula holder (56).

6. Cannula according at least any of the preceding claims,
**wherein**
any unwanted rotation of the said unwandle (18) relative to the said cannula holder (16) is prevented by a conveniently adapted projection (38) extending from the said cannula holder (16) and engaging, at least by sections, in a linear axial recess in the said handle (18).

7. Cannula according preferably to any of the preceding claims,
**wherein**
the said protector cap (82) is provided on its outer wall with at least one axially extending, hollow cylindrical pocket (84, 85) for receiving the used cannula (12).

8. Cannula according to claim 7,
**wherein**
at least one channel-like recess (92, 94) extends from the end face (40) of the said protector cap (82) and terminates in the said pocket (84, 86).

9. Cannula according to any of the preceding claims,

**wherein**

the said cannula holder (16) comprises a portion (98) extending towards the cannula tip which, regarded from the side of the cannula tip, is located in front of a predetermined breaking point (28) and the said handle (13).

## Revendications

1. Canule (10,46, 52, 80) en particulier jour effectuer une ponction dans un récipient comme pour traitement extracorporel au sang tel qu'une dialyse ou une plasmaphorèse avec une pointe de canule (14) affûtée obliquement et un support de canule (16, 56) recevant la canule (12), sur lequel on peut enfiler un tuyau (60) à l'arrière et qui est entouré du moins dans une zone par une poignée (18) constituée de préférence à la manière d'une aile et avec un chapeau de protection (20) entourant la pointe de la canule, canule **caractérisée en ce que**

le chapeau de protection (20) et la poignée (18) peuvent être poussés comme unité de la pointe de l'aile (14) sur le support de canule (16, 56), en ce que le support de canule présente un collet d'encliquetage (48, 62) et la poignée présente une rainure d'encliquetage (50, 64) correspondant à ce collet et en ce que le collet d'encliquetage est encliqueté dans la rainure d'encliquetage dans le cas de l'unité disposée sur le support de canule et en ce que le support de canule (16, 56) présente du côté du tuyau un gradin (34, 58).

2. Canule selon la revendication 1,
**caractérisée en ce que**

le support de canule (16) présente à une certaine distance de son extrémité arrière une collerette (34), contre lequel on peut appuyer le tuyau.

3. Canule salon la revendication 2,
**caractérisée en ce que**

la zone (22) de la poignée (18) entourant le support de canule (16) repose sur la face de la collerette (34) tournée du côté opposé au tuyau.

4. Canule selon au moins l'une des revendications précédentes,
**caractérisée en ce que**

le support de canule (16, 56) et/ou la poignée (18) ou sa section (22) entourant le support de canule présentent des saillies d'encliquetage ou des éléments d'encliquetage (36, 38, 62, 64, 66, 68, 48, 50).

5. Canule selon au moins l'une des revendications précédentes,
**caractérisée en ce que**

la poignée (18) repose sur une butté (70) partant du support de canule (56) de préférence dans la zone tournée vers la pointe de canule (40) et en ce que dams le sens axial du support de canule (56) partent de la butée des ergots d'arrêt (72) et/ou des encoches d'arrêt, qui interagissent avec des encoches d'arrêt partant de la zone (22) du côté frontal entourant le support de canule (56).

6. Canule selon au moins l'une des revendications précédentes,
**caractérisée en ce que**

la poignée (18) présente pour empêcher une torsion par rapport au support de canule (16) un évidemment de forme linéaire s'étendant axialement (36), dans lequel au moins une saillie (38) ajustée partant du support de canule (16) vient en prise au moins par zone.

7. Canule selon de préférence l'une des revendications,
**caractérisée en ce que**

le chapeau de protection (82) présente du côté extérieur de la paroi une cavité de réception (84, 86) en forme de cylindre creux s'étendant axialement pour receuillir la canule (12).

8. Canule selon la revendication 7,
**caractérisé en ce que**

partant de la zone frontale (40) du chapeau de protection (82) s'étend au moins un renfoncement (92, 94) de forme annulaire, qui débouche dans la cavité de réception (84, 86).

9. Canule selon au moins l'une des revendications précédentes,
**caractérisé en ce que**

le support de canule (16) est pourvu d'une section (98) s'étendant en direction de la pointe de la canule, section qui passe, en tenant compte de la pointe de la canule, devant le point destiné à la rupture (28) et la poignée (18).

Fig. 1

Fig. 2

48

40        20        28

46        Fig. 3        28

Rippen um 90° versetzt gezeichnet

18

22

16

34

20        14

Einzelheit X

Fig. 4

48

28    50

Fig. 5

Fig. 6

Fig.7

Fig. 8

Fig.9

Fig. 10

Fig.11